(19)

Europäisches Patentamt

European Patent Office

Office européen des brevets

(11) **EP 3 284 401 A1**

(12) **EUROPEAN PATENT APPLICATION**

(43) Date of publication:
**21.02.2018 Bulletin 2018/08**

(21) Application number: **17169144.7**

(22) Date of filing: **02.05.2017**

(51) Int Cl.:
*A61B 5/00* (2006.01)  *A61B 5/11* (2006.01)
*A61B 5/024* (2006.01)  *A61B 7/02* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **02.05.2016  HU 1600288**

(71) Applicant: **Szegedi Tudományegyetem**
**6720 Szeged (HU)**

(72) Inventors:
• **SIPKA, Gábor**
**6726 Szeged (HU)**
• **FIDRICH, Márta**
**6725 Szeged (HU)**
• **SZABÓ, Tibor**
**6728 Szeged (HU)**
• **NAGY, Tamás**
**6724 Szeged (HU)**
• **BILICKI, Vilmos**
**6725 Szeged (HU)**
• **BÁRTFAI, György**
**6726 Szeged (HU)**

• **BITÓ, Tamás**
**6720 Szeged (HU)**
• **MINGESZ, Róbert**
**6727 Szeged (HU)**
• **GINGL, Zoltán**
**6725 Szeged (HU)**
• **GYIMÓTHY, Tibor**
**6726 Szeged (HU)**
• **VADAI, Gergely**
**6724 Szeged (HU)**
• **MAKAN, Gergely**
**6721 Szeged (HU)**
• **VÁNYA, Melinda**
**6724 Szeged (HU)**
• **JAKÓ, Mária**
**6722 Szeged (HU)**
• **ZÖLEI-SZÉNÁSI, Ráhel**
**6723 Szeged (HU)**
• **BORBÁS, János**
**6721 Szeged (HU)**

(74) Representative: **Harangozo, Gabor**
**Danubia**
**Patent & Law Office LLC**
**Bajcsy-Zsilinszky út 16**
**1051 Budapest (HU)**

(54) **METHOD, DEVICE AND COMPUTER PROGAM PRODUCT FOR EXAMINATION OF INTRA-UTERUS CONDITION OF THE FOETUS**

(57)    During the method concerning the examination of the intra-uterine condition f the foetus, the mechanical vibrations induced by the heart of the foetus, together with other vibrations coming from the body of the expectant mother, are sensed by the at least one electro-mechanical transformer placed on the body surface of the expectant mother, the detected vibrations are transformed into electrical signals; the electrical signals so received are led to the signal processing units and processed, during which the electrical signals are pre-amplified by manually adjustable amplification of automatic amplification control; the frequencies including the heart sound of the expectant mother are removed from the amplified electrical signals of the perceived vibrations by active band filtering; the signal level reduction due to band filtering is compensated for by amplification; the filtered signal is subjected to further transformation before processing, so that it is modulated to a carrier frequency in the range of 5.5-6.5 kHz, and led to the signal processing unit that way; in the signal processing unit, first the modulated signal is demodulated, then it is processed.
   A device suitable for implementing the method comprises the following main components:
- an elastic belt that can be fixed on the abdomen of the expectant mother;
- at least one electro-acoustic transducer (22) and filtering and signal conditioning grade (22) detecting mechanical vibration placed in the belt, connected to a communication device (21), preferably a smart phone, preferably through wireless, e.g. Bluetooth, connection;
   Between the electro-acoustic transducer (22) and the communication device (21), a band filter is inserted, that is

**(Cont. next page)**

designed as band filter cutting off sharply the frequencies of under around 20 Hz and above 200 Hz, and 70 Hz and 300 Hz, respectively, including also the heart sound of the expectant mother.

The computer program product to be run on the device, e.g. the smart phone, processes the measured signals, displays them visually, and makes proposals and shows trends to the user based on the processed signals.

Start

Acquiring signals — 1

Providing the signal — 2

Pre-amplifying the signal — 3

Filtering the signal — 4

Amplifying the signal — 5

Modulating the signal — 6

Demodulating the signal — 7

Analyzing and evaluating — 8

Displaying the result — 9

**Fig. 1**

**Fig. 2**

**Description**

The present invention relates a method for examination of the intra-uterine condition of the foetus according to the preamble of claim 1, as well as a device according to the preamble of claim 24, enabling telemonitoring of foetal heart sounds via a smartphone based on phonocardiography measurements.

Detecting foetal movement and foetal heart rate is of outstanding importance, since periodic control can reduce foetal perinatal disease and mortality and, consequently, the physical, mental and psychological load on the pregnant mother.

The heart function of the healthy, well-fed foetus, well-oxygenated by the placenta is typical in the last month of pregnancy, its rate is between 120 and 160 heartbeat/minute. Deviation from that is considered abnormal, and requires other examinations or maybe intervention. Consequently, to prevent complications and for the sake of early diagnosis, it is worthwhile to monitor the foetal heart sound regularly.

The examination of the variability of the foetal heart rhythm provides useful information on the condition of the central nervous system of the foetus. Heart rhythm changes are usually examined over a 20-30-minute interval, taking into account the sleep-wakefulness periods of the foetus.

In clinical pregnant care practice, there are two options for examining the heart of the foetus. One is ultrasound examination conducted in the 18th to 21st weeks of pregnancy, when the anatomic structure of the heart and the large vessels are examined. So-called cardio-tocographic (abbreviation: CTG) examinations are made from the 38th week on (in case of pregnancy with complication, even from the 24th week). CTG registers foetal heart frequency (cardio) and the contractions of the myometrium (toco) jointly, based on ultrasound technology.

The importance of foetal movement counting and of checking the heart function of the foetus keeps increasing from Week 24 on. In current practice, movement is observed during manual ultrasound examination performed by the physician. It is also possible for the pregnant woman to indicate the movements she perceives during CTG by push-button.

Various solutions are known for the acoustic sensing of the foetal heart sound and the determination of the instantaneous value of the heart rhythm, to draw inferences as to the intra-uterine condition, development, exposition to risk of the foetus. The slamming of the foetal heart valve carries extremely low energy; that energy as sound wave must go through the chest of the foetus, the amniotic fluid, the uterine muscles and the abdominal wall of the pregnant mother, and that is the sound wave that makes the skin surface move, that is to be detected besides other, undesired, sounds.

Today, the most widespread examination method is the foetal Doppler ultrasound that is applied to examine foetal circulation, heart function, frequency, blood circulation by an ultrasound device. There exist already ultrasound devices developed and distributed for home use. However, it is not recommended to use them for regular, e.g. daily, monitoring, because the ultrasound testing method is invasive, that is, actively intervening, and adverse effects correlating with frequent use cannot be excluded.

Several efforts have been made to find a non-invasive alternative to the ultrasound devices for the examination of the foetus. Such devices include e.g. foetal electrocardiography (abbr. fECG), foetal magneto-cardiography (abbr.: fMCG) or foetal phonocardiography (abbr.: fPCG). These are all of the passive kind. However, fECG is highly positioning-dependent and requires many electrodes. fMCG is big and expensive. These are all hindrances to long-term home monitoring.

Various methods are known for the acoustic sensing of foetal heart sound, and the determination of the instantaneous value of the heart rhythm - to draw inferences as to the intra-uterine condition of the foetus and its exposition to risk. There are devices that can listen to the foetal heartbeat and store records. These typically use either an external device or simply the microphone e.g. of a smartphone to record heart sound. Generally speaking, these are entertainment-type applications, devices featuring sophisticated design, sometimes with extra functions, e.g. pregnancy wheel, horoscope forecast.

There are many commercially available electronic stethoscopes, but the foetal heart sound can be heard poorly or not at all through them, since they are optimised for the frequency of the adult heart sound. The decisive majority of known devices developed for home use, applying the Doppler technology, are not recommended for monitoring taking place several times a day or in the long term.

US 20110098555 A1 discloses an acoustic medical device, method and medical examination and diagnostic device for auscultation based on phonocardiography, abbr. PCG, measurement, associated with a mobile phone application. It provides no guidance for fixing the stethoscope or for continuous monitoring.

HU 1100254 A1 concerns on the one hand a method and on the other a device for the evaluation of the phonocardiography (PCG) signal typical of the foetal heart function, where a digital signal is produced by digitising the measured analogue phonocardiography signal; an identification step is performed, where valve sounds present in the digital signal, determining heart beats, are identified, and confidence values are assigned to the valve sounds; a first classification step is performed, where the heart beats are assigned, based on the confidence value assigned to one of the valve sounds associated with them, to a noiseless class and a noisy class; a digital signal cleared of noise is defined from the digital signal by retaining the heart beats assigned to the noiseless class; a modelling step is performed, where the parameters of at least one function modelling at least one valve sound by heartbeat of the digital signal cleared of noise are determined; and a foetal heart function assessment step is performed based on the modelling functions defined in the modelling step. The proposed method is meant to provide a complex solution; it is applicable also for filtering out certain heart function disorders in gestational age that cannot be realised easily under simpler, e.g. home, conditions, and neither is the information being supplied adjusted to the needs and preparedness of the users; the large-size device is not suitable for monitoring under normal way-of-life conditions and it is rather expensive.

Thus it can be said that there is a real market gap between the consumer expectations and the known solutions and products.

We have therefore set the target of developing a small-size, cheap, simple, non-invasive device suitable for home use and the associated mobile application designed for the acoustic detection of foetal heart sound and the determination of the instantaneous value of the heart rhythm, and with which monitoring can be realised effectively also by a lay person.

Our objective in this context is the accurate and reliable detection of the foetal heart sound in the home environment that can provide medically relevant information and make proposals to the pregnant mother e.g. to contact a physician. A device capable of implementing a new method, of an affordable price, to be used comfortably and simply, without medical assistance, is needed, with which the user, that is, most often the pregnant mother, can record foetal movement and uterine contraction that can provide information that is also medically relevant. Our objective is to ensure comfortable wear, and to detect the heart sound and measure the heart rhythm over even a longer period, continuously.

Our objective is, moreover, to make it possible also to analyse trends, rhythmic changes, population dynamics through the device.

The task being set has been solved on the one hand by a method for the examination of the intra-uterine condition of the foetus according to the features of claim 1, as well as by a device according to the features of claim 24. The novelty of the invention lies in the novel-type hardware- and software-based noise filtering: the disturbing or irrelevant signal components are separated from the measured signal, and only the signal range to be subjected to examination is retained and transferred to a separate evaluation unit by a frequency-modulating technique, preferably by a wireless protocol. From the heart sounds, noises, recorded on the abdomen wall of the pregnant mother, the heart sounds of the pregnant mother and the foetus are separated by a method based on spectral separation, the noises are filtered out, which makes it possible to reliably determine the pulse rate of the foetus.

Preferred variations of the invention have been formulated in dependent claims.

The invention will be presented in more detail via a possible realization, taken in conjunction with the accompanying drawings, wherein:

Figure 1            shows a flowchart of an exemplary implementation of the method according to the invention,

Figure 2            shows an exemplary structure of the sensor electronics,

Figure 3            shows an exemplary structure of the measuring electronics,

Figure 4            shows the frequency spectra of signals derived from the foetal and the maternal heart sounds,

Figures 5a and 5b   show a distance between the foetal heart and the detector head at occiput posterior and occiput anterior position, respectively,

Figure 6            shows the S1 and S2 foetal heart sound frequencies changing during pregnancy, and

Figure 7            shows an exemplary structure of the communication device.

Reference will now be made to the exemplary embodiments illustrated, and specific language will be used herein to describe the same. It will nevertheless be understood that no limitation of the scope of the invention is thereby intended.

Before the present invention is disclosed and described, it is to be understood that this invention is not limited to the particular structures, process steps, or materials disclosed herein, but is extended to equivalents thereof as would be recognized by those ordinarily skilled in the relevant arts. It should also be understood that terminology employed herein is used for the purpose of describing particular embodiments only and is not intended to be limiting.

Phonocardiography, known under the abbreviation of PCG, is a non-invasive, observational (passive) examination method. Measuring takes place through the abdominal wall of the pregnant mother, at one or several sampling points. PCG is one of the earliest-used low-cost heart-function-testing methods: today's PCG devices are the upgraded versions of the initial stethoscope, then phonendoscope. However, PCG had been forced to the background for a long time by the current advanced ultrasound and CTG techniques. In our days, the use of the PCG is spreading again, for example in the field of telemedicine, thanks to the extensively wide spread of smartphones.

In case of phonocardiography, i.e. PCG-based, signal processing, the basis for all signal processing methods is provided by Joachim Nagel's publication of 1986, "New diagnostic and technical aspects of foetal phonocardiography". fPCG has obvious advantages since it is cheap, safe and makes long-term monitoring at home feasible.

PCG devices detect sounds originating from the mechanical operation of the heart, in the 0.1-1000 Hz range, detected usually by pressure sensor operated by mechanical vibration - phono-acoustically. The various organs and tissues conduct, modulate, the mechanical vibrations of the heart differently, and that is how they reach the external skin surface of the body where the vibrations and waves are detected by mechanical acoustic and/or pressure sensors. Mechanical vibrations will be transformed first into analogue then into digital signals in the detector head filled with gas and in the tube.

It is an essential feature of the method and device according to the invention that they should be capable of determining foetal heart frequency by the phonocardigraphic, i.e. PCG, method. To do that, the foetal heart sound needs to be filtered out reliably from among the other incoming signals. The signal concerned is to be transformed into an input signal that can be interpreted by the evaluation devices, primarily smartphones, then into data and, finally, into relevant and reliable information for the user and, as the case may be, the medical staff. Of course, these functions do not replace medical diagnosis and therapy; the device does not constitute a medical device.

The method starts out from the fact that the acoustic signal of the foetal heart sound is an approximately periodic signal, with a frequency spectrum and amplitude that changes in time, close to noise level in the majority of cases in terms of signal level. The heart sound signal must be selected from among the detected signals based on some criterion that is valid for every signal coming from foetal heart sound.

The useful signals originating from the heart sound can be separated from the other signals by examining the frequency spectrum of the detected signals, that is, the signal we look for among the detected signals has a frequency pattern corresponding to the spectrum of the systolic component being a first component of the heart sound. The separation by frequency necessary for that is done by an active bandpass filter that can be applied most advantageously in the low frequency ranges. The most important practical requirement to be met by the filter circuits is to have the most even possible transmission in the pass-band, the sharpest cut in the blocking range and that phase shift in the pass-band should be the linear function of the frequency.

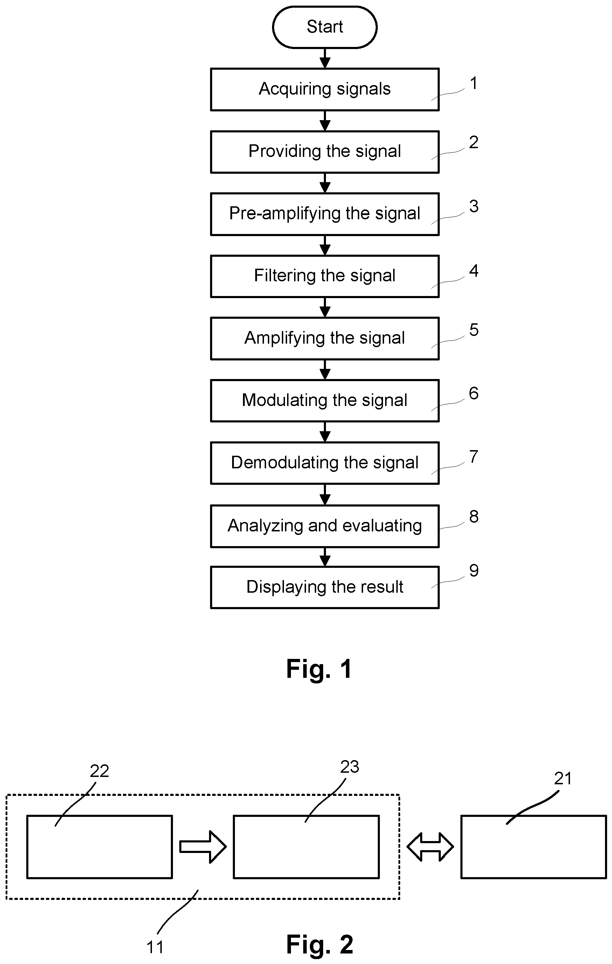

As can be seen in Figure 1, in Step 1, a signal or, more precisely, signals are detected in the manner mentioned in the foregoing. The device implementing the method may have dedicated ports, but for example if a smartphone is used, as recommended, in most cases it has no such ports, so the measurement signals are inputted through its own interface that is expediently a wireless interface. In a preferred embodiment, electrets microphone is used for the measurement, but as will be obvious for those skilled in the art, it is also possible to use an electro-acoustic detector, i.e. phonendoscope head, or phetoscope acoustic resonator or a detection network consisting of several phonendoscope detection heads, the structure and operation of these devices being known to those skilled in the art. Digitisation of the analogue signal takes place already in the evaluation unit, e.g. smartphone, in the way that is normal there. The detected acoustic signal appears transformed into electrical signal by the acoustic sensor or detector or network as unfiltered, unamplified signal that is transferred for pre-processing in Step 2. This signal is preamplified in Step 3, then in Step 4 the pregnant woman's heart sound frequency of less than 20-25 Hz is filtered out, so that in occiput anterior (back of the skull forward) position, the frequency band under 20 Hz and over 200 Hz, and in occiput posterior (back of the skull in the back) position under 70 Hz and over 300 Hz is cut off. This signal is amplified again in Step 5, then frequency-modulated by 6000 Hz modulation frequency in Step 6. This frequency-modulated, mixed, signal is sent to the interface of the evaluation unit, e.g. smartphone, or the input of a separate mike if there is one, and it is demodulated in Step 7, analysed and evaluated in Step 8 according to the measures indicated in the following Table 1 and, finally, the results are displayed in Step 9.

Table 1

| Feature (Name, sign) | Indication | Narrative | calculation ( <= 1 minute PCG registration) | Typical values |
|---|---|---|---|---|
| Interval between heartbeats | RR | intervals between heartbeats, also called RR intervals | Time interval(s) between heartbeats (RRi-ith RR interval) | 0.5-0.375 s |

(continued)

| Feature (Name, sign) | Indication | Narrative | calculation ( <= 1 minute PCG registration) | Typical values |
|---|---|---|---|---|
| **Normal frequency** | FHR v. BFHR [1/minut e] | *Instantaneous heart frequency (1 minute):*<br><br>60 divided by the interval between two heartbeats in s. | $f_i = 60/RR_i$<br><br>(This is conditional on being able to perceive the exact time of the heartbeat, on having a specific time that the device can detect, and on being able to measure/calculate continuously over the interval between the two.) | normal value: 120-160/minute<br><br>Value given based on the section where no acceleration or deceleration occurs and the difference between the fastest and the slowest section is max. 25 beat/minute. |
| | | *Average heart frequency (per 1 minute):* Average of instantaneous heart frequencies over a period of one minute | $$f_{mean} = \frac{\sum_{i=1}^{n} \dfrac{60}{RR_i}}{n-1}$$<br>where *n* is the number of RR intervals. | |
| **Variability or oscillation** | FHRV or VorO [1/minut e] | *Instantaneous variability:* By variability we mean the interval between consecutive heart actions *Average variability:* Average of instantaneous variability over a period of one minute | $$\frac{\sum_{i=1}^{n} RR_i - RR_{i+1}}{n-1}$$<br>time difference between consecutive heart actions | tachycardia-(>160/minute) bradycardia-(<120/minute) |
| **Variability amplitude** | A [1/minut e] | Its amplitude is the difference of the highest and lowest heart frequency over one minute.<br>You look for the last registered one-minute period and, and deduct from the highest FHR value the lowest one. | $\max(f_i) - \min(f_i)$<br><br>(over a one-minute CGT section without periodic changes) | Normal value: 5-15/minute. |
| **Variability frequency** | Df [1/minut e] | Number of cycles occurring over one minute. (How many times the FHR curve intersects the virtual baseline frequency line) When you have the baseline frequency, you can observe how many times the instantaneous frequency value is measured consecutively first under then above, or vice versa, the baseline frequency. | piece(i), where $f_i$ - FHR = 0 and $f'(i) \neq 0$ | Normal: 3-6/minute. |

(continued)

| Feature (Name, sign) | Indication | Narrative | calculation ( <= 1 minute PCG registration) | Typical values |
|---|---|---|---|---|
| Transitory frequency increase | ACC [1/minut e] | Short section of 10-20 seconds, where the heart frequency increases by at least 10/minute. Sensing 10/minute change in the increase of foetal heart frequency, the given section is regarded as acceleration until the time of its return to the baseline | Frequency increment up to 10-20 s | A = 10-30/minute |
| Transient frequency decrease | DEC [1/minut e] | Frequency decrease of a few seconds, at a rate of at least 10/minute | Frequency decrease lasting for 10-20 s | A = 10-30/minute |

Basic foetal heart rate, foetal movement, maternal uterine contraction are medically significant examination features. The signal behind the band-pass filter comprises together the maternal and the foetal heart sounds that are separated by the known Fourier spectrum calculation, considering that the foetal heart rhythm is approximately twice that of the maternal heart rhythm.

Foetal heartbeat variability and oscillation (fHRV) are calculated from the demodulated, band-passed signal, as indicated in Table 1 for a possible embodiment.

To examine foetal movement, the pregnant mother can indicate the point in time when movement begins, and with that the signal section typical of movement can be delimited in the examined signal. This makes it possible to perform the statistical analysis of the movement patterns. Under special circumstances, the pregnant mother-foetus communication can be examined, for example with the help of amplifiers placed in a flexible belt fixing the device on the body, to examine for example movement occurring under the effect of musical sound.

In a preferred embodiment, sensors placed in a flexible belt make not only one-off, but also continuous 24-hour examination feasible. In this case, the examination of the foetal heart sound can be started at any time on the initiative of the pregnant mother. The individual measurements and their results get stored, one by one, in the smartphone, or they can be uploaded to a server in case of network connection. In the actual recording, if a certain event takes place, the pregnant mother can initiate in retrospect the saving of an expediently min. 30-minute time window where the event to be examined occurred.

After the evaluation of the psycho-physiological signals recorded during the representation or display performed in Step 9, it is possible to notify the physician, i.e. send an SMS or an e-mail, if one or several alert thresholds had been crossed. Thus for instance foetal heart rhythms that are permanently low - bradycardia, i.e. foetal heart frequency of less than 120/minute - or permanently high - tachycardia, i.e. foetal heart frequency in excess of 160/minute - can be signalled. In case of continuous monitoring or examination of at least 15-30 minutes, the basic rhythm of the heart (fHRV) is analysed as well, that makes it possible to identify the movement types and sleeping status, where faster and slower heart rhythm makes movement and sleep, respectively, likely, and through which sleep-wakefulness-movement periods can be identified. Special movements can also be examined during the measurement of heart rhythm deviating from the basic rhythm (e.g. while listening to music).

Motion tests can be analysed by examining the number and quantity of movements over a given interval. In weeks 38-41, load tests can be made by awakening the foetus. In case of continuous monitoring, periodic foetal heart rhythm changes associated with contractions can also be analysed on the basis of the periodically recorded data.

The device according to the invention that makes the execution of the proposed method feasible, presented here exclusively as preferred example, comprises two main units: an elastic belt that can be fixed on the abdomen of the pregnant woman and that hosts as can be seen in Figure 2 the sensing, measuring, signal processing and communication electronics, and thus constitutes a measuring unit 11 that can be placed and fixed on a body, and an evaluation and display unit, independent of measuring unit 11, in wireless communication connection with it, that is constituted by a communication device 21, in the presented case in a particularly advantageous way a so-called smartphone, and a computer program product in it which, when running, makes the evaluation and display unit execute the evaluation-display part of the method reviewed in connection with Figure 1, so that the latter becomes capable of the further processing of the measured signals, of their visual display, of making proposals and showing trends based on the processed signals. The communication electronics of the measuring unit 11 arranged in the belt that can be fastened on the body is capable of some kind of known wireless communication adjusted to the communication device 21, that is, the evaluation and display unit ever; in case of the embodiment presented here, Bluetooth communication is applied, the structure and functioning mechanism of which is well-known to those skilled in the art.

Figure 2 shows a possible embodiment of the phonocardiography device; it can be divided into three parts, the first two of which are preferably treated as a compact measuring unit 11. A smartphone, mentioned already in connection with the embodiment being presented here, constitutes the communication device 21 and, similarly to that, all units can be operated by (rechargeable) battery and are portable as is known to persons skilled in the art, so we have a device that is easy to use.

The measuring unit 11 comprises an electro-acoustic transducer stage 22 that is in the presented case a converted stethoscope that is sufficiently sensitive in the above-indicated frequency range. The output of the electro-acoustic transducer stage 22 is connected to the input of a filtering and signal conditioning stage 23 that performs amplification, signal filtering and signal conditioning in the monitored frequency range.

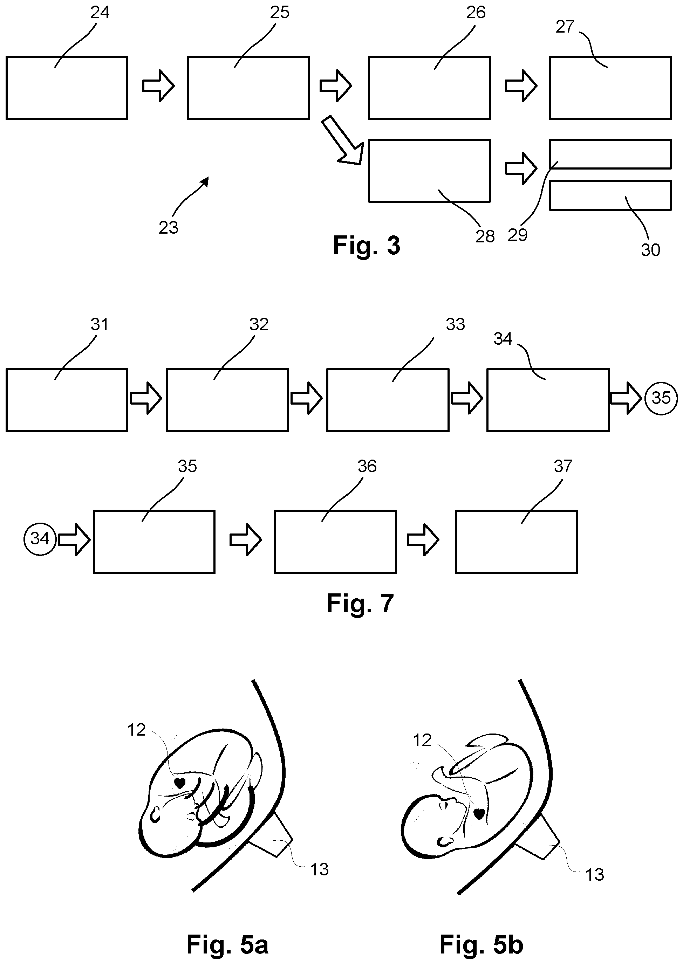

Figure 3 shows the components of the filtering and signal conditioning stage 23. These include a pre-amplifier 24, a bandpass filter 25, an amplifier 26 and a frequency modulator 27, connected one after the other electrically as listed. For the sake of the ideal positioning of electro-acoustic transducer stage 22 on the abdominal wall of the pregnant woman, the device is designed so as to make it capable of audibly indicating the foetal heartbeats, if requested. Therefore, in a preferred embodiment, at least one of a loudspeaker 29 or an earphone 30 is connected to the output of a power amplifier 28 connected to the output of the bandpass filter 25.

The pre-amplifier 24 comprises two separate amplifier stages that should have automatic gain control or manually adjustable gain for the sake of easier use. The pre-amplifier 24 comprises a pre-amplifier stage constructed of an operational amplifier connected to an electrets microphone applied as acoustic sensor and, furthermore, a mike amplifier with automatic gain control. The latter is a most essential part of the pre-amplifier, since the foetal heartbeat, falling into a narrow frequency range, is very faint. The structure and operation of the stages mentioned above are known to those skilled in the art.

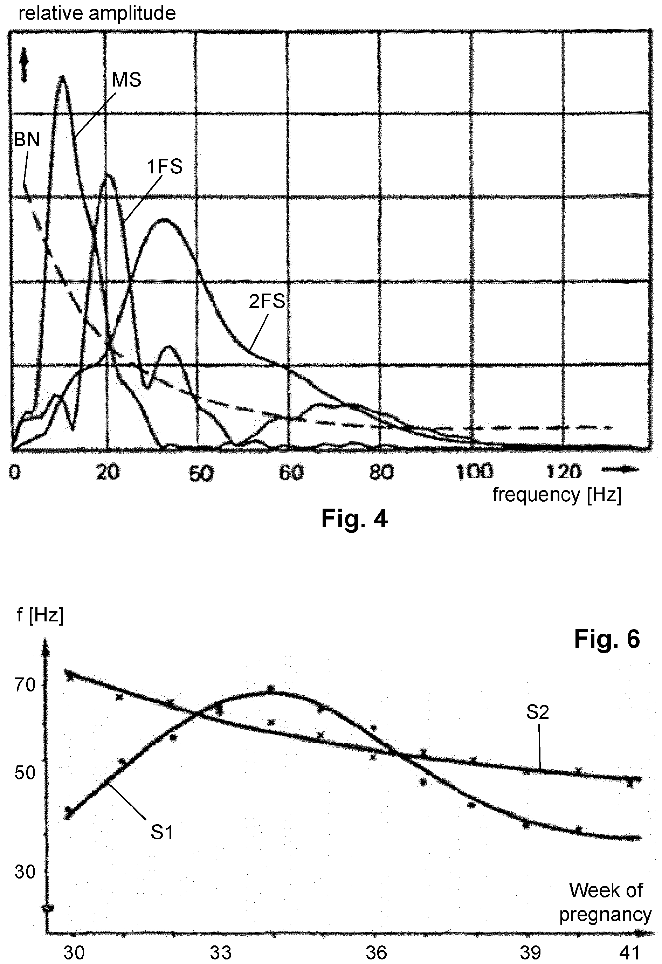

The disturbing effect of the noise of the maternal heartbeats needs to be removed, filtered out of the measured signal, to make the foetal heartbeat well-measurable. The typical, known, frequency spectra of the signals originating from the foetal and the maternal heartbeats, respectively, are shown in Figure 4. In the figure, it is possible to observe the changes of the maternal heart sound MS, the first foetal heart sound 1FS, the second foetal heart sound 2FS and the background noise BN. Filtering out the frequency under 20-25 Hz of the maternal heartbeat is ensured by active bandpass filter 25 cuts the frequency ranges under 20 Hz and above 200 H, and under 70 Hz and above 300 Hz, respectively.

The foetal heartbeat frequency depends on the position of the foetus: an occiput posterior position - resulting in a signal of higher frequency - and an occiput anterior position - resulting in a signal of lower frequency - are distinguished. Figures 5a and 5b show the relative distance between the foetal heart 12 and the detector head 13 used for measuring in occiput posterior and anterior position, respectively. According to a preferred embodiment of the invention, two band-pass filters are applied to measure the heartbeats of the foetus in these two positions, designed as active, inverting, narrow band-pass filter of 20-200 Hz and 70-300 Hz in occiput anterior and posterior position, respectively, that is an eighth-order Chebyshev-type band-pass filter with multiple negative feedback, with 0.01 dB typical dampening fluctuation in the transmission range.

The foetal heartbeat spectra change during pregnancy. Figure 6 shows the typical frequencies of foetal heart sounds S1 and S2 in function of time. From Week 34 on, the peaks shift towards the lower frequencies. The above-mentioned filtering circuits have been designed in consideration of this known fact, because that makes the frequency spectrum chosen by us suitable for detecting the foetal heart sound from Week 30 on throughout the period of pregnancy.

The output of the bandpass filter 25 is connected in the present example to the input of a further controllable amplifier 26.

The smartphones proposed for utilisation filter and condition the microphone signals in different ways depending on their type. Therefore, to forward the signals to a smartphone, we have inserted a frequency modulator 27, in consideration of the fact that the modulated signal needs to be demodulated by the software in the smartphone afterwards. The signals are modulated preferably to a carrier frequency in the spectrum of 5.5-6.5 kHz.

According to a preferred embodiment and the one that is presented here, the filtering and signal conditioning stage 23 comprises a power amplifier 28 and an acoustical display that displays the foetal heart sounds audibly, facilitating to a large extent the placement of the acoustic sensor on the abdomen wall of the pregnant woman, i.e. the quick identification of the so-called punctum maximum. To do that, the loudspeaker 29 and/or earphones 30 may be connected to the output of the power amplifier 28 connected to the output of the bandpass filter 25.

Figure 7 shows schematically the elements, relevant for the invention, of a communication device 21 applied to perform the evaluation. The program running on the smartphone or a PC used as an alternative, carrying out the method, provides for real-time signal processing on the one hand and for the evaluation of the registered FHR values, making it possible thereby to monitor the heart rhythm changes. The signal received from the communication electronics is demodulated via the known, own communication unit of the smartphone by a demodulator 31; and the signal is stored in an intermediary storage medium 32 connected to the latter's output; the data to be investigated are taken out by a peak-detector stage 33 connected to the latter's output; the data sought for are disambiguated by a frequency-defining stage 34 connected to the latter's output, the medically relevant at least one piece of information is defined by a processing stage 35 connected to the latter's output, the information is stored in the memory of the smartphone or a connected external data storage device (e.g. SD card) by a storage stage 36 connected to the latter's output, and it is sent, as the case may be, by the own communication unit of the smartphone to a dedicated remote server, and the specified information is displayed by a display stage 37, also the own visual and/or acoustic unit of the smartphone, connected to the latter's output.

The structure and functioning mechanism of the demodulator 31 and the intermediary storage medium 32 are known to those skilled in the art, so their detailed presentation is not necessary here. Cross-correlation between the incoming signal and a pre-recorded and stored short foetal heartbeat sample is calculated in peak detector 33. The energy associated with the points of the correlated signal are received e.g., by a Teager energy operator known to those skilled in the art from the work of Kvedalen, Eivind entitled "Signal processing using the Teager energy operator and other nonlinear operators" Master University of Oslo Department of Informatics 21, 2003. The heartbeats appear on the energy curve of the correlated signal as peaks, easy to detect by level-intersecting algorithm.

Foetal heart rate (FHR) is determined by the frequency-defining stage 34 so that the foetal heart rate curve associated with the recording is obtained by subtracting the time of a respective previous heartbeat from the time of the heartbeats obtained during peak detections.

The processing stage 35 is used to define the baseline, calculate the variability of the heartbeat, i.e. oscillation, amplitude, and look for heart frequency acceleration and deceleration. One can also record special circumstances of the heartbeat recording here, e.g. listening to music, presence of the father; set periodic heartbeat recordings that can be stored for reference, e.g. 20-minute measurement made at 06:00 a.m.; do continuous heart sound monitoring, within which the actual recording can be saved within a given time window; awaken the sleeping foetus by a vibration function - although that is a so-called load test recommended only for pregnant mothers in weeks 38-41.

In case of continuous monitoring, the basic rhythm of the heart is analysed by the processing stage 35, where slower heart rhythm is likely to mean sleep and faster heart rhythm movement. Inferences can be drawn as to the sleep-wakefulness-movement periods; periodic foetal heart rhythm changes associated with contractions, recorded regularly by the pregnant mother, can be analysed. Conclusions can be drawn as to the special movement of the foetus upon measuring a heart rhythm that is different from the basic one e.g. when listening to music, in the presence of the father, and the movements of the foetus, e.g. the quantity of movement over a given time period, can also be analysed.

The recordings and other data to be recorded are stored by the storage stage 36, of known structure and operation mechanism, and said storage stage 36 ensures data retrieval and sharing.

The display stage 37 provides for intuitive, smooth use by the user. It is possible to display a guide for placement, to provide feedback if the recording is not adequate, e.g. only the heartbeat of the pregnant mother can be detected or the background noise is excessive, for example, a Short Message Service, in short SMS, can be sent about temporary abrupt heart rhythm decline, permanently low (bradycardia: foetal heart frequency under 120/minute) or permanently high (tachycardia: foetal heart frequency in excess of 160/minute) foetal heart rhythm.

Although not indicated in the figure, those skilled in the art will understand that the device can also be designed so as to make the pregnant mother able to indicate subjectively by a handling element, e.g. by operating a push-button, the movement of the foetus and the contractions.

The safety of the exemplary device embodying the method according to the invention is to be given high priority: the power supply of the electronic circuits is provided by (rechargeable) batteries, and the whole device is electrically insulated, so that neither the pregnant mother, nor the foetus can be exposed to any electric effect. The device can be provided with the known polarity protection, with battery charge indicator, and with protection against splashing water.

Let us mention among the main advantages of the method according to the invention that it is suitable for home use, and makes it possible to conduct observation without the presence of a professional, even over a longer time. Its advantages include, furthermore, raising the chances of live birth, providing reassuring and reliable feedback to the pregnant mother and the physician, respectively, on the status of the foetus; easy and effective use by the pregnant mother and the physician, respectively; alarm in case of complication; exclusion of false alarm in the same context; fast feedback and, last but not least, ease of wearing that does not hinder movement. Since the device used for the method is non-invasive (PCG-based) and provides for easy wear, heartbeat detection and heart rhythm measurement can take place over a longer period, continuously.

It is possible to conduct measurements for tens of minutes, to obtain medically relevant information from the measured data, based on which feedback can be provided on information characterising the condition of the foetus - inferences concerning the special movement of the foetus upon the measurement of heart rhythm differing from the basic one, inference as to the daily sleep-wakefulness-movement period - on the basis of which the condition of the foetus be assessed in a reliable way. The measurement results can be stored and searched, that may be a great advantage for medical diagnostics; they can be included optionally into the decision-making process of the specialist physician.

The phonocardiography method requires no professional assistance, keeping in mind that obtaining a signal of adequate quality depends on the relative position of the detection head and the foetal heart. Since the examination of the foetus is completely passive, the foetus is not subject to any energy radiation or other external effect, the examination can be conducted for as long time and as frequently as desired, preferably at home, in a monitoring-type of way.

Based on the regular measurements, the method provides for the analysis of trends, rhythmic changes. Collecting the data so received makes it feasible to analyse population dynamic or launch other medical research.

List of used reference signs:

1-9 step
11 measuring unit
12 foetal heart
13 detector head
21 communication device
22 electro-acoustic transducer stage
23 filtering and signal conditioning stage
24 pre-amplifier
25 bandpass filter
26 amplifier
27 frequency modulator
28 power amplifier
29 loudspeaker
30 earphone
31 demodulator
32 intermediary storage medium
33 peak detection stage
34 frequency-defining stage
35 processing stage
36 storage stage
37 display stage

**Claims**

1. A method for the examination of intra-uterine condition of a foetus, the method comprising:

   - sensing, by at least one sensor placed on a body surface of a pregnant mother, mechanical vibration induced by the heart of the foetus in the uterus, together with other vibrations coming from the body of the pregnant mother;
   - transforming the perceived vibration into an electrical signal;
   - transmitting the electrical signal so received to a signal processing unit and processing the transmitted electrical signal;
   - during the signal processing, pre-amplifying said electrical signals with a manually adjustable gain or by automatic gain control;
   - removing the frequencies including the heartbeat of the pregnant mother from the amplified electrical signals of the perceived vibration by active bandpass filtering;
   - compensating for a signal level reduction caused by said bandpass filtering by further amplification;
   - subjecting the filtered signal to further transformation before evaluation by modulating said filtered signal to a carrier frequency in the range of 5.5-6.5 kHz and transmitting the modulated filtered signal for further evaluation;
   - performing evaluation by first demodulating, then processing said modulated signal; and
   - displaying acoustically and/or optically said evaluated signal to a user.

2. The method according to claim 1, *characterised in that* performing evaluation comprises defining mechanical vibration induced by the heart of the foetus as foetal heart frequency with the help of a peak detecting algorithm.

3. The method according to claim 2, ***characterised in that*** performing evaluation comprises defining a baseline, calculating heart variability based on signal oscillation and signal amplitude, and looking for heart frequency acceleration and deceleration.

4. The method according to any of claims 1-3, ***characterised by*** further comprising recording the movements of the foetus during the evaluation.

5. The method according to any of claims 1-4, ***characterised by*** further comprising recording contractions during the evaluation.

6. The method according to any of claims 1-5, ***characterised in that*** performing evaluation further comprises defining the predefined circumstances of the heartbeat recording considered special in advance.

7. The method according to any of claims 1-6, ***characterised by*** further comprising performing said heartbeat recordings in a scheduled way.

8. The method according to claim 7, ***characterised by*** further comprising storing said heartbeat recordings made in a scheduled way for reference.

9. The method according to any of claims 1-8, ***characterised by*** further comprising waking up the sleeping foetus by vibration in the case of pregnant mothers in Weeks 38-41.

10. The method according to any of claims 1-9, ***characterised by*** further comprising performing the heartbeat recordings continuously, and saving an actual recording within a predefined time window.

11. The method according to claim 10, ***characterised by*** further comprising emitting a warning signal in case of sudden temporary heart rhythm decline.

12. The method according to claim 11, ***characterised in that by*** further comprising emitting said warning signal independently or as supplement to a short text message, SMS.

13. The method according to claim 11, ***characterised by*** further comprising emitting a warning signal in case of permanently low foetal heart rhythm.

14. The method according to claim 13, ***characterised by*** further comprising emitting said warning signal in case of bradycardia, i.e. foetal heart frequency of less than 120/minute.

15. The method according to claim 10, ***characterised by*** further comprising emitting a warning signal in case of permanently high foetal heart rhythm.

16. The method according to claim 15, ***characterised by*** further comprising emitting said warning signal in case of tachycardia, i.e. foetal heart frequency in excess of 160/minute.

17. The method according to any of claims 10-15, ***characterised by*** further comprising analysing a basic rhythm of the heart based on continuous monitoring.

18. The method according to any of claims 10-17, ***characterised by*** further comprising inferring sleep-wakefulness-movement periods of the foetus from the continuous monitoring.

19. The method according to claim 11, ***characterised by*** further comprising analysing the foetal movements based on the quantities of movement over a predefined period.

20. The method according to claim 5, ***characterised by*** further comprising analysing the periodic foetal heart rhythm changes associated with contractions.

21. The method according to claim 10, ***characterised by*** further comprising inferring special movement of the foetus from detecting heart rhythm differing from the basic rhythm.

22. The method according to claim 1, *characterised by* further comprising providing feedback if it is determined during signal processing that the recording is inadequate.

23. The method according to claim 1, *characterised by* further comprising filtering out the frequencies including also the heartbeat of the pregnant mother depending on the position of the foetus, cut-off spectra of the frequencies to be filtered out being the one under 20 Hz and above 200 Hz for occiput posterior position, and under 70 Hz and above 300 Hz for occiput anterior position.

24. A device for the examination of the intra-uterine condition of the foetus, comprising
    at least one electro-acoustic transducer (22) sensing mechanical vibration,
    a portable multifunctional communication device (21),
    where the at least one electro-acoustic transducer (22) comprising a device that can be fastened adjacent to the uterus on the body of the pregnant mother, and
    the electro-acoustic transducer (22) is connected to the communication device (21) by one of a wired or wireless connection, furthermore
    where the communication device (21) is provided with at least one of an acoustical and optical display unit,
    furthermore, the communication device (21) comprises a signal evaluating unit,
    where the signal evaluating unit comprising a unit evaluating and displaying input data based on the stored instructions,
    *characterised in that*
    a filtering and signal conditioning stage (23) is inserted between the electro-acoustic transducer (22) sensing mechanical vibration and the portable multi-functional communication device (21);
    the filtering and signal conditioning stage (23) comprises a pre-amplifier (24) provided with manual or automatic gain control, the output of which is connected to the input of a bandpass filter (25), the output of the latter is connected through an amplifier (26) to the input of the frequency modulator (27) where the bandpass filter (25) comprising an active band pass filter (25) cutting off sharply the frequencies of around under 20 Hz and above 200 Hz and under around 70 Hz and above 300 Hz comprising also the heartbeat of the pregnant mother.

25. The device according to claim 24, *characterised in that* the input of a power amplifier (28) is connected to the output of the bandpass filter (25), and the output of the former is led to the input of a loudspeaker (29) or earphone (30).

26. The device according to claim 24 or 25, *characterised in that* the measuring unit (11) is arranged on an elastic belt that can be placed on the body of the pregnant mother.

27. The device according to any of claims 24-26, *characterised in that* the measuring unit (11) is connected to the communication device (21) by wired connection.

28. The device according to any of claims 24-26, *characterised in that* the measuring unit (11) is connected to the communication device (21) by a short-range wireless protocol.

29. The device according to any of claims 24-28, *characterised in that* the frequency modulator (27) connected to the output of the amplifier (26) after the bandpass filter (25) is realised as stage modulating the signal led to it to the carrier frequency in the 5.5-6.5 kHz spectrum.

30. Computer program product that can be executed by a computer, comprising program instructions that, when cause a communication device (21) to undertake the steps of the method according to any of Claims 1-23.

Start

Acquiring signals ———— 1

Providing the signal ———— 2

Pre-amplifying the signal ———— 3

Filtering the signal ———— 4

Amplifying the signal ———— 5

Modulating the signal ———— 6

Demodulating the signal ———— 7

Analyzing and evaluating ———— 8

Displaying the result ———— 9

# Fig. 1

22    23    21

11

# Fig. 2

**Fig. 3**

**Fig. 7**

**Fig. 5a**          **Fig. 5b**

relative amplitude

MS

BN

1FS

2FS

0  20  50  60  80  100  120

frequency [Hz]

**Fig. 4**

f [Hz]

**Fig. 6**

70

50

30

S2

S1

Week of
pregnancy

30  33  36  39  41

Europäisches
Patentamt

European
Patent Office

Office européen
des brevets

**EUROPEAN SEARCH REPORT**

Application Number

EP 17 16 9144

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 524 631 A (ZAHORIAN STEPHEN A [US] ET AL) 11 June 1996 (1996-06-11)<br>* column 3, line 62 - column 5, line 30 *<br>* column 8, line 24 - line 49 *<br>----- | 1-20 | INV.<br>A61B5/00<br>A61B5/11<br>A61B5/024<br>A61B7/02 |
| X | WO 2012/061827 A2 (WEST WIRELESS HEALTH INST [US]; ROHAM MASOUD [US]; SALDIVAR ENRIQUE [U) 10 May 2012 (2012-05-10)<br>* the whole document *<br>----- | 1-20 | |
| X | US 2006/229518 A1 (OFEK ERAN [IL])<br>12 October 2006 (2006-10-12)<br>* abstract *<br>* paragraph [0012] - paragraph [0013] *<br>* figure 1 *<br>----- | 1-20 | |
| X | WO 96/15713 A1 (INST OF RESPIRATORY MEDICINE L [AU]; SULLIVAN COLIN EDWARD [AU]) 30 May 1996 (1996-05-30)<br>* abstract *<br>----- | 1-20 | TECHNICAL FIELDS SEARCHED (IPC) |
| X | US 5 140 992 A (ZUCKERWAR ALLAN J [US] ET AL) 25 August 1992 (1992-08-25)<br>* abstract *<br>----- | 1-20 | A61B |

The present search report has been drawn up for all claims

| Place of search | Date of completion of the search | Examiner |
|---|---|---|
| The Hague | 11 January 2018 | Van Dop, Erik |

EPO FORM 1503 03.82 (P04C01)

Europäisches
Patentamt

European
Patent Office

Office européen
des brevets

Application Number

EP 17 16 9144

---

## CLAIMS INCURRING FEES

The present European patent application comprised at the time of filing claims for which payment was due.

[X] Only part of the claims have been paid within the prescribed time limit. The present European search report has been drawn up for those claims for which no payment was due and for those claims for which claims fees have been paid, namely claim(s):

16-20

[ ] No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

[ ] All further search fees have been paid within the fixed time limit. The present European search report has been drawn up for all claims.

[ ] As all searchable claims could be searched without effort justifying an additional fee, the Search Division did not invite payment of any additional fee.

[ ] Only part of the further search fees have been paid within the fixed time limit. The present European search report has been drawn up for those parts of the European patent application which relate to the inventions in respect of which search fees have been paid, namely claims:

[ ] None of the further search fees have been paid within the fixed time limit. The present European search report has been drawn up for those parts of the European patent application which relate to the invention first mentioned in the claims, namely claims:

[ ] The present supplementary European search report has been drawn up for those parts of the European patent application which relate to the invention first mentioned in the claims (Rule 164 (1) EPC).

**EP 3 284 401 A1**

## ANNEX TO THE EUROPEAN SEARCH REPORT
## ON EUROPEAN PATENT APPLICATION NO.

EP 17 16 9144

This annex lists the patent family members relating to the patent documents cited in the above-mentioned European search report.
The members are as contained in the European Patent Office EDP file on
The European Patent Office is in no way liable for these particulars which are merely given for the purpose of information.

11-01-2018

| Patent document cited in search report | | Publication date | Patent family member(s) | | Publication date |
|---|---|---|---|---|---|
| US 5524631 | A | 11-06-1996 | NONE | | |
| WO 2012061827 | A2 | 10-05-2012 | AU | 2011323102 A1 | 23-05-2013 |
| | | | BR | 112013010900 A2 | 04-10-2016 |
| | | | CA | 2816894 A1 | 10-05-2012 |
| | | | EP | 2635191 A2 | 11-09-2013 |
| | | | JP | 2014500742 A | 16-01-2014 |
| | | | KR | 20140035313 A | 21-03-2014 |
| | | | US | 2012232398 A1 | 13-09-2012 |
| | | | WO | 2012061827 A2 | 10-05-2012 |
| US 2006229518 | A1 | 12-10-2006 | NONE | | |
| WO 9615713 | A1 | 30-05-1996 | AT | 237988 T | 15-05-2003 |
| | | | DE | 69530523 D1 | 28-05-2003 |
| | | | DE | 69530523 T2 | 18-03-2004 |
| | | | EP | 0843530 A1 | 27-05-1998 |
| | | | JP | H10511015 A | 27-10-1998 |
| | | | WO | 9615713 A1 | 30-05-1996 |
| US 5140992 | A | 25-08-1992 | NONE | | |

For more details about this annex : see Official Journal of the European Patent Office, No. 12/82

EPO FORM P0459

18

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20110098555 A1

- HU 1100254 A1

**Non-patent literature cited in the description**

- **JOACHIM NAGEL.** *New diagnostic and technical aspects of foetal phonocardiography,* 1986

- **KVEDALEN ; EIVIND.** Signal processing using the Teager energy operator and other nonlinear operators. Master University of Oslo Department of Informatics, 2003, vol. 21